**Europäisches Patentamt**

**European Patent Office**

**Office européen des brevets**

(19)

(11) Veröffentlichungsnummer : **0 168 380**
**B1**

(12) **EUROPÄISCHE PATENTSCHRIFT**

(45) Veröffentlichungstag der Patentschrift :
**07.09.88**

(21) Anmeldenummer : **85890135.8**

(22) Anmeldetag : **24.06.85**

(51) Int. Cl.⁴ : **A 61 M 16/00**, A 61 M 16/20

(54) **Gerät zur Lungenbehandlung von Menschen oder Tieren.**

(30) Priorität : **29.06.84 AT 2111/84**

(43) Veröffentlichungstag der Anmeldung :
**15.01.86 Patentblatt 86/03**

(45) Bekanntmachung des Hinweises auf die Patenterteilung : **07.09.88 Patentblatt 88/36**

(84) Benannte Vertragsstaaten :
**BE CH DE FR GB IT LI LU NL SE**

(56) Entgegenhaltungen :
**DE-A- 2 152 376**
**DE-A- 3 126 207**
**DE-A- 3 227 463**
**DE-B- 2 238 928**

(73) Patentinhaber : **Czech, Kurt, Dr.**
**Schwarzenbergplatz 13**
**A-1040 Wien (AT)**

(72) Erfinder : **Czech, Kurt, Dr.**
**Schwarzenbergplatz 13**
**A-1040 Wien (AT)**

(74) Vertreter : **Wolfram, Gustav, Dipl.-Ing.**
**Schwindgasse 7 P.O. Box 205**
**A-1041 Wien (AT)**

## Beschreibung

Die Erfindung betrifft ein Gerät zur Lungenbehandlung von Menschen oder Tieren.

Bekannte Geräte dieser Art — sogenannte Jet-Generatoren — arbeiten mit einem 2/2-Weg-Ventil, d. h. einem Ventil mit zwei Anschlüssen und zwei Schaltstellungen, also einem einfachen Öffner-Schließer. Als Ventile wurden unterschiedliche Absperrorgane, wie Magnetventile, pneumatische Ventile oder magnetisch vorgesteuerte Ventile eingesetzt.

Diese Geräte werden u. a. zur künstlichen Beatmung oder zur therapeutischen Behandlung von Sekretansammlungen im Lungen- oder Bronchialraum verwendet.

In vielen Anwendungsfällen hat es sich als besonders zweckmäßig herausgestellt, die von einem solchen Gerät erzeugten, etwa 50 bis 200 ms dauernden Druckstöße der Spontanatmung oder einer künstlichen Beatmung zu überlagern, wobei beispielsweise zähe, thixotrope Sekretansammlungen verflüssigt werden und leicht abgehustet werden können. Auch die Sauerstoffaufnahme durch verletzte Lungenflügel — beispielsweise bei Vorliegen von Serienrippenbrüchen — kann durch Verwendung der Geräte außerordentlich verbessert werden.

Die Applikationsstelle kann sich an einem Endotrachealtubus befinden; die Leitung kann aber auch in einem Mundstück enden, wobei die Mundhöhle die Applikationsstelle ist. Die Geräte arbeiten mit verhältnismäßig hohem Gasdruck und stellen in bekannten Ausführungsformen ein Gefährdungsrisiko dar, da während der Öffnungsphase des Ventils eine direkte Verbindung zwischen Druckgasquelle und Applikationsstelle besteht. Wenn aber die Druckverbindung in die Lunge bzw. in die Trachea — z. B. infolge Hängenbleibens des Ventils—längere Zeit, auch nur etwa 1 oder 2 s, bestehen bleibt, führt dies zu mitunter irreversiblen Barotraumen. Nur ganz kurze Druckstöße, eben im Bereich von etwa 50 bis 200 ms sind für den Patienten unschädlich und begründen den gewünschten therapeutischen Effekt.

Wegen dieses Risikos hat man verschiedene Anstrengungen unternommen, um die Funktion der Ventile sicherer zu gestalten, so z. B. ein Magnetventil nicht als federbelastetes Ventil auszubilden, sondern für jede Schaltstellung ein eigenes Solenoid vorzusehen. Weiters hat man Drehschieberventile in Betracht gezogen. Es sind auch Nachschaltdruckwächter vorgeschlagen worden.

Ein weiterer Nachteil der bekannten Geräte liegt in der von diesen gelieferten Rechtecksform der Druckstöße, wodurch bei jeder Öffnungsphase des Ventils relativ große Gasvolumina zur Applikationsstelle strömen. Wird ein Patient künstlich beatmet, können diese Gasmengen sogar zu erheblichen Störungen der Beatmungsgeräte führen.

Aus der DE-A-2 152 376 ist ein Verfahren bzw. eine Einrichtung zur Aufrechterhaltung und Unterstützung der Atmung bekannt, wobei drei verschiedene Arten des Strömungsverlaufes des einzuatmenden Gases erzielbar sind, nämlich ein sogenannter (zeitlich) « konstanter », ein « zunehmender » und ein « abnehmender » Strömungsverlauf. Bei allen diesen Strömungsarten muß das Gas über ein Hauptsteuerventil in einer gemeinsamen Leitung strömen.

Hier ist kein höherfrequent betätigbares Ventil vorgesehen, sondern lediglich ein mit Atemfrequenz gesteuertes Hauptventil, welches im geöffneten Zustand hängen bleiben kann, so daß, da während der Einatmungsphase der Patient mit der Druckgasquelle direkt verbunden ist, ebenfalls die Gefahr des Entstehens von Barotraumen besteht.

Die Erfindung bezweckt die Vermeidung der geschilderten Nachteile und Schwierigkeiten und stellt sich die Aufgabe, ein Gerät der eingangs beschriebenen Art zu schaffen, welches betriebssicherer ist als die bisherigen, eine zweckmäßigere und effektivere Impulsform der Druckstöße liefert und die Patienten zuverlässig vor Schäden bewahrt.

Ein weiteres Ziel der Erfindung besteht darin, die an der Applikationsstelle einzubringenden, kurzzeitigen Gasstöße in regelbarer Weise zu befeuchten.

Die gestellte Aufgabe wird mit einem Gerät nach Anspruch 1 gelöst.

Ein Gerät mit einem Jet-Generator, welches mit einem Ventilator zur intermittierenden positiven Druckbeatmung kombiniert ist, ist aus der DE-A-3 227 463 bekannt. Dieses Gerät weist einen Druckkessel auf, der zur Steuerung der Druckstoßfrequenz dient und dessen Inhalt sich nicht in den Patienten entleert. Während der aktiven Phase besteht ein direkter Weg zwischen der Druckgasquelle und dem Patienten über ein 3/2-Weg-Ventil, wodurch die Gefahr des Entstehens von Barotraumen besteht.

Bei dem erfindungsgemäßen Gerät hingegen besteht somit zu keinem Zeitpunkt eine direkte Verbindung zwischen Druckgasquelle und Applikationsstelle. Das Volumen des Einzelgasstoßes entspricht maximal dem Inhalt des Druckkessels. Bei plötzlichen Gebrechen an den Ventilen oder an der Taktsteuerung ist eine Volumsüberladung des Patienten und somit eine Druckschädigung der Lunge ausgeschlossen.

Die Impulsform der Druckstöße ist je nach Taktlänge angenähert trapez- oder dreieckförmig. Daraus resultiert eine trotz hohem Spitzengasfluß niedrige Gasmenge pro Einzeltakt, verglichen mit der während eines von herkömmlichen Geräten gelieferten Druck- bzw. Gasstoßes von Rechteckform ausströmenden Gasmenge.

Durch geeignete Wahl des Strömungswiderstandes, welcher von Querschnitt und Länge des vom passiven 3/2-Weg-Ventil zur Applikationsstelle führenden Leitungsstückes bestimmt wird, kann der dreieck- bzw. trapezförmige Impulsverlauf weitgehend der Form einer Sinushalbwelle angenähert werden. Eine solche Impulsform ist auf anderem Weg nur durch aufwendige und klinisch kaum einsetzbare Kolben-Pleuel-Aggregate erzielbar.

Zweckmäßig sind als aktives 3/2-Weg-Ventil ein Magnetventil mit einem Dichtkolben von geringer Masse und als passives 3/2-Weg-Ventil ein Schnellentlüftungsventil mit im Vergleich zu dem aktiven 3/2-Weg-Ventil größeren Strömungsquerschnitten vorgesehen. Ein solches aktives 3/2-Weg-Ventil mit kleinen Strömungsquerschnitten kann problemlos auch mit hohen Frequenzen betrieben werden.

Auch der Einsatz eines Ventiles anderen Typs, wie eines elektropneumatischen Ventils, als aktives Ventil ist möglich.

So ist nach einer weiteren bevorzugten Ausführungsform als aktives Ventil ein pneumatisches Ventil vorgesehen, dessen Steuereingang über ein weiteres aktives pneumatisches 3/2-Weg-Ventil mit der Druckgasquelle verbindbar ist, wobei der Steuereingang des weiteren aktiven pneumatischen 3/2-Weg-Ventiles mittels einer Gasleitung, in der sich gegebenenfalls ein regelbares Drosselorgan befindet, zwischen dem aktiven 3/2-Weg-Ventil und dem passiven 3/2-Weg-Ventil an die Gaszuführungsleitung angeschlossen ist.

Vorzugsweise ist auch bei dieser Ausführungsform als passives 3/2-Weg-Ventil ein Schnellentlüftungsventil mit im Vergleich zu den beiden aktiven pneumatischen 3/2-Weg-Ventilen größeren Strömungsquerschnitten vorgesehen.

Da bei einem passiven Ventil der Dichtkörper nur geringe Masse aufweist, vermag ein solches Ventil trotz größerer Strömungsquerschnitte auch höherfrequenten Druckstößen leicht zu folgen.

Zum Betrieb eines erfindungsgemäßen Gerätes in der voranstehend beschriebenen Ausführungsform, bei welcher die Steuerung bzw. Betätigung des aktiven 3/2-Weg-Ventiles durch ein weiteres pneumatisches 3/2-Weg-Ventil erfolgt, ist kein elektrischer Anschluß erforderlich. Diese Bauform eignet sich daher ganz besonders auch für einen mobilen Einsatz, beispielsweise in Rettungsfahrzeugen oder Feldlazaretten ohne Stromversorgung.

Medizinische Gase müssen befeuchtet werden. Dies gilt insbesondere für Gase aus Druckleitungen und Druckflaschen, wo sie aus technischen Gründen nahezu wasserfrei sein müssen. Die erforderliche Wassermenge liegt in der Größenordnung von 40 mg/l. Schnelle Gasströme wie bei Jet-Generatoren können nicht nach den herkömmlichen Methoden, wie Durchleiten durch erwärmtes Wasser, Überleiten knapp über eine erwärmte Wasseroberfläche, Vorbeistreichen an dochtartigen Konstruktionen, befeuchtet werden. Eine Befeuchtung vor dem Ventil — also im Bereich langsamer Gasströmung — ist zwar technisch möglich, erfordert aber aus hygienischen Gründen wegen des Keimwachstums auf feuchten Oberflächen eine Sterilisation des gesamten Systems. Eine Befeuchtung der Gasstöße wurde bisher apparativ sehr aufwendig durch Wasserzusatz mittels aktiver Dosiereinrichtungen (z. B. Rollerpumpen) realisiert.

Das Befeuchtungsproblem ist somit bisher nicht zufriedenstellend gelöst.

Wird eine nach dem Injektorprinzip (Parfumzerstäuber) arbeitende Befeuchtungsvorrichtung, bei der an der Mündung der Gasleitung ein Flüssigkeitsansaugrohr endigt, verwendet, so wird, wenn der Durchmesser des Wasserzuführungsrohres etwa gleich groß ist wie die Mündung der Gasleitung, zu viel Feuchtigkeit mitgerissen und gewissermaßen ein Nebel gebildet, wogegen bei einem Flüssigkeitsansaugrohr mit einem zu dünn gewählten Innendurchmesser kein Wasser einbringbar ist.

Dieses Problem wird bei einer vor der Applikationsstelle am Ende der Gaszuführungsleitung angeordneten, nach dem Injektorprinzip arbeitenden Befeuchtungsvorrichtung besonders zweckmäßig dadurch gelöst, daß das Ansaugrohr der Befeuchtungsvorrichtung einen etwa gleichen inneren Durchmesser hat wie die Gaszuführungsleitung, jedoch im Bereich der Mündung in die Befeuchtungsflüssigkeit eine Drosselstelle, insbesondere ein gegen die Innenwand des Ansaugrohres abgedichtetes Kapillarrohr, aufweist.

Diese Befeuchtungsvorrichtung arbeitet selbstansaugend, ohne daß komplizierte, außenliegende Zusatzvorrichtungen, wie peristaltische Pumpen, vorgesehen werden müssen. Durch geeignete Wahl des Innendurchmessers und der Länge der Drosselstelle kann die Menge der jeden Gasstoß zugesetzten Feuchtigkeit ganz einfach geregelt werden. Trotz minimalen Bauaufwandes arbeitet die Befeuchtungsvorrichtung mit für klinische Zwecke ausreichender Genauigkeit. Der entscheidende Vorteil liegt in der Handlichkeit und einfachen Sterilisierbarkeit dieser Zusatzvorrichtung.

Nach einer bevorzugten Ausführungsform des erfindungsgemäßen Gerätes ist im Druckkessel ein Druckfühler eingebaut, welcher über ein elektronisches Steuerelement elektrisch leitend mit dem aktiven 3/2-Weg-Ventil verbunden ist. Bei Erreichen des vorgewählten Solldruckes im Druckkessel wird das aktive 3/2-Weg-Ventil durch ein vom Druckfühler abgegebenes Signal umgeschaltet, bei einem Magnetventil wird beispielsweise durch einen Stromimpuls die Stromzufuhr zu der Magnetspule des aktiven Ventils unterbrochen und dadurch die Entleerung des Druckkessels zur Applikationsstelle hin eingeleitet. Bedingt durch die mechanische Trägheit der Ventile kommt es zwischen der Druckerfassung durch den Druckfühler und dem effektiven Ansprechen der Ventile zu einer Zeitverzögerung, welche in den meisten Fällen im Bereich von etwa 30 ms liegt. Da der Druckanstieg im Druckkessel keine lineare Funktion der Zeit, welche zur Kesselfüllung zur Verfügung steht, ist, führt die erwähnte, annähernd konstante Zeitverzögerung zu verschieden großen Überschreitungen des Solldruckes, je nachdem, ob der Solldruck niedrig oder knapp submaximal ist.

Vorzugsweise umfaßt aus diesen Gründen das elektronische Steuerelement einen Mikroprozessor zur exakten Regelung des Umschaltzeitpunktes des aktiven 3/2-Weg-Ventils unter Berücksichtigung der in Abhängigkeit vom Solldruck im Druckkessel veränderlichen Druckzunahme während der konstanten Zeitspanne zwischen der Druckerfassung durch den Druckfühler und dem Ansprechen der beiden 3/2-Weg-Ventile.

Als Druckfühler ist insbesondere ein piezoelektrischer Druckwandler in den Druckkessel eingebaut.

In der klinischen Praxis ist es in vielen Fällen sehr erwünscht, ein Gasgemisch von genau definierter Zusammensetzung anzuwenden. Mischapparaturen zur Erzielung von beispielsweise Luft-Sauerstoffgemischen in medizinischen Geräten arbeiten meist als Analogmischer mit verstellbaren Nadeldüsen. Derartige Geräte lassen sich relativ klein bauen, sie sind aber nahe den beiden Extrempunkten (fast reiner Sauerstoff oder fast reine Luft) ziemlich ungenau.

Da bei intermittierender Strömung für kurze Zeit undefinierte Strömungszustände herrschen, können derartige Systeme bei Jet-Generatoren überdies nur unter Zwischenschaltung großer Pufferkessel mit ausreichender Genauigkeit verwendet werden.

Herkömmliche Mischvorrichtungen für Beatmungsgeräte gehen von der Voraussetzung aus, daß das gewünschte Mischungsverhältnis in jedem Augenblick eingehalten sein muß. Es ist zwar eine Mischvorrichtung mit digitaler Mischung bekannt, welche Vorrichtung die Einhaltung exakter Mischungsverhältnisse auch in den erwähnten Extrembereichen erlaubt, doch enthält auch diese Mischvorrichtung einen mehrere Liter fassenden Mischkessel.

Zur Überwindung der beschriebenen Unzulänglichkeiten ist nach einer weiteren Ausgestaltung des erfindungsgemäßen Gerätes vorgesehen, daß zur Beimischung eines zweiten Gases in der Gaszuführungsleitung in Richtung der Gasströmung gesehen vor dem aktiven 3/2-Weg-Ventil ein weiteres aktives 3/2-Weg-Ventil und zwischen den beiden aktiven 3/2-Weg-Ventilen ein Reduzierventil eingeschaltet sind, wobei die beiden Eingänge des weiteren aktiven 3/2-Weg-Ventils jeweils mit einer Druckgasquelle verbunden sind.

Die einzelnen Gasstöße werden nach dieser Ausgestaltung abwechselnd mit dem einen und mit dem anderen Gas abgegeben ; das Taktverhältnis wird dabei so eingestellt, daß die gewünschte Gasmischung resultiert, wobei von der Erkenntnis ausgegangen wird, daß die Lunge immer ein genügend großes Gasvolumen enthält, um selbst als Mischkessel zu fungieren. Besonders vorteilhaft ist bei dieser Ausführungsform weiters, daß auf die Höhe des Druckes der in das weitere aktive 3/2-Weg-Ventil einströmenden Gase nicht besonders geachtet zu werden braucht, da der Gasdruck mittels des Reduzierventiles auf bestimmte Höhe beschränkt wird. Die beiden Gase können auch mit stark voneinander abweichenden Drücken zugeführt werden, ohne daß die Mischgenauigkeit des Gerätes nachteilig beeinflußt würde.

Vorzugsweise sind die beiden aktiven 3/2-Weg-Ventile über ein elektronisches Steuerglied miteinander elektrisch verbunden, welches eine Umschaltung des weiteren aktiven 3/2-Weg-Ventils nur zuläßt, so lange das erste aktive 3/2-Weg-Ventil die Gasströmung vom Reduzierventil unterbricht. Ein Umschalten des weiteren aktiven 3/2-Weg-Ventils von einem Gas auf das andere, so lange das erste aktive 3/2-Weg-Ventil noch für die Gaszufuhr von der Druckgasquelle geöffnet ist, stört die Funktion des passiven 3/2-Weg-Ventils.

Die Erfindung wird im folgenden anhand der Zeichnung noch näher erläutert.

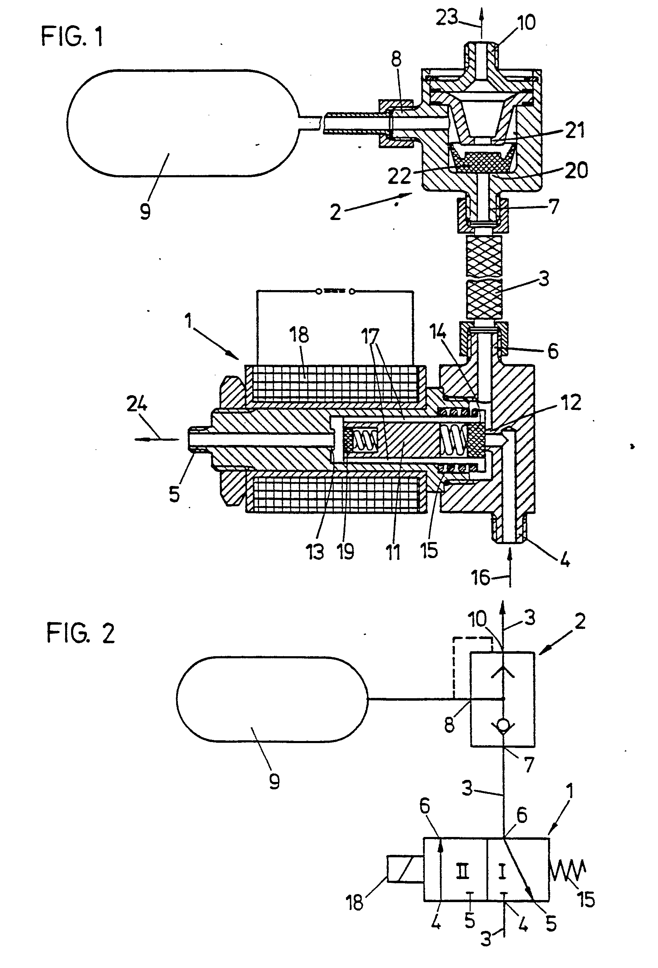

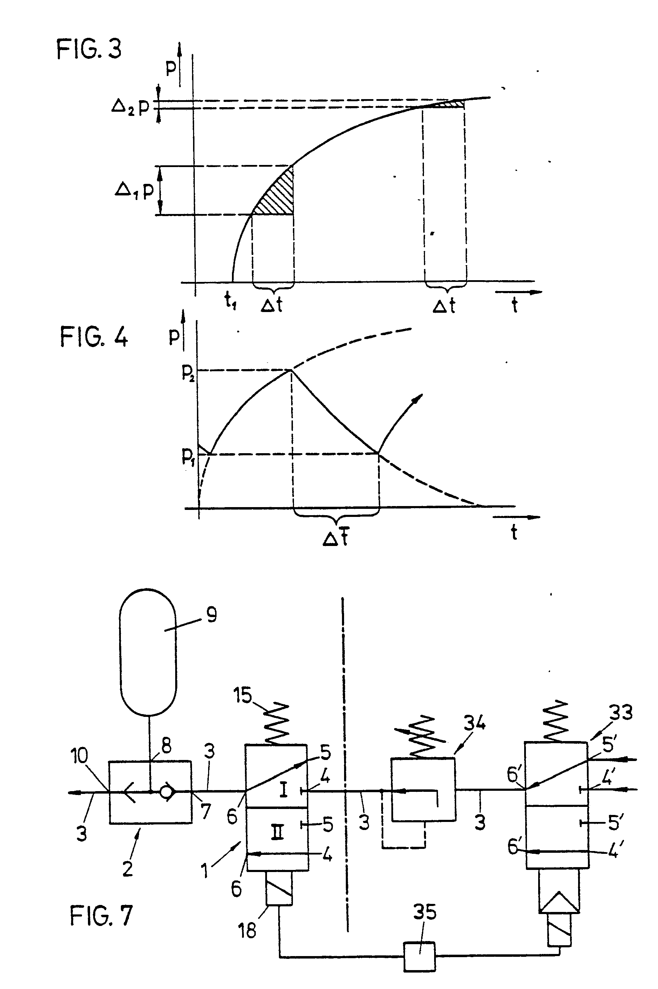

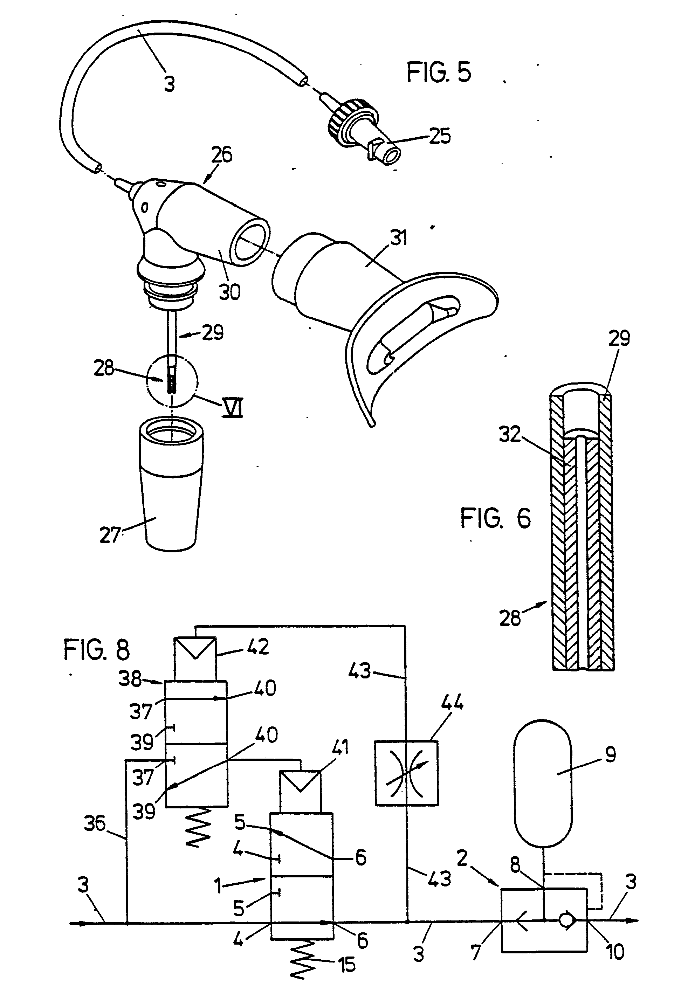

Fig. 1 zeigt schematisch eine Ausführungsform des erfindungsgemäßen Gerätes, teilweise im Schnitt, wobei die Druckquelle und die Applikationsstelle nicht dargestellt sind. In Fig. 2 ist der Schaltplan des Gerätes nach Fig. 1 gezeigt, wobei für die Ventile Schaltsymbole nach DIN ISO 12 19 eingesetzt wurden. In Fig. 3 ist der Druckverlauf im Druckkessel in Abhängigkeit von der Anschlußzeit an die Druckgasquelle gezeigt. Aus Fig. 4 ist der tatsächliche Druckverlauf im Kessel bei einem Betriebszustand zur Bildung höherfrequenter Druckstöße ersichtlich. In Fig. 5 sind die einzelnen Teile einer zum erfindungsgemäßen Gerät gehörenden Befeuchtungsvorrichtung auseinandergenommen einschließlich dem vom passiven 3/2-Weg-Ventil wegführenden Leitungsteil und einem Mundstück dargestellt. Fig. 6 ist eine Schnittdarstellung eines vergrößerten Ausschnittes vom Mündungsbereich des Flüssigkeitsansaugrohres der Befeuchtungsvorrichtung nach Fig. 5. In Fig. 7 ist ein Schaltplan einer zur Beimischung eines zweiten Gases geeigneten Ausführungsform des Gerätes dargestellt und Fig. 8 veranschaulicht den Schaltplan einer Ausführungsform des erfindungsgemäßen Gerätes mit einem pneumatischen Ventil als aktives 3/2-Weg-Ventil.

Ein aktives 3/2-Weg-Ventil — und zwar ein Magnetventil — ist in Fig. 1 allgemein mit 1 bezeichnet, und ein passives 3/2-Weg-Schnellentlüftungsventil mit 2. Die Ventile 1 und 2 sind in eine von einer Druckgasquelle zur Applikationsstelle führende Leitung 3 eingeschaltet, wobei in Fig. 1 nur das die beiden Ventile miteinander verbindende Gasleitungsstück, welches ein Metallrohr oder ein flexibler Schlauch aus druckfestem Material sein kann, dargestellt ist. Die Gaszuführungsleitung von der Druckgasquelle führt zum Anschluß 4 des aktiven Ventils 1. Der Anschluß 5 vom Ventil 1 mündet in einen unter vergleichsweise niedrigem Druck stehenden Behälter oder in die umgebende Atmosphäre. Der Anschluß 6 vom Ventil 1 ist mit dem Anschluß 7 des passiven Ventils 2 verbunden. Die zweite, mit 8 bezeichnete Anschlußstelle des Ventils 2 mündet in einen Druckkessel 9. Der dritte Anschluß 10 des passiven Ventils 2 führt zur Applikationsstelle.

Der Dichtkolben 11 vom Ventil 1 ist zwischen den beiden Ventilsitzen 12 und 13 hin- und herbewegbar. In einer ersten Schaltstellung wird die Dichtung 14 des Dichtkolbens 11 unter Wirkung der Feder 15 gegen den Ventilsitz 12 gepreßt, wodurch der von der Druckgasquelle aus der Richtung des Pfeiles 16 kommende Gasstrom unterbrochen wird. Die Anschlüsse 5 und 6 des aktiven Ventils 1 stehen gleichzeitig über Ausnehmungen 17. im Dichtkolben 11 miteinander in Verbindung. In der zweiten Schaltstellung, nämlich bei Stromfluß durch die Magnetspule 18, wird die Dichtung 19 des Dichtkolbens 11 gegen den Ventilsitz 13 gepreßt, wodurch eine Strömungsverbindung zwischen den Anschlüssen 4 und 6 des Ventils 1 hergestellt und der Anschluß 5 gesperrt wird.

Der zwischen den Ventilsitzen 20 und 21 hin- und herbewegbare Dichtkörper 22 des passiven Ventils 2 stellt entweder eine Verbindung zwischen dem Anschluß 7 des Ventils 2 — und somit der Druckgasquelle — und dem Anschluß 8, d. h. dem Druckkessel 9, oder zwischen dem Anschluß 8 und dem Anschluß 10, also zwischen dem Druckkessel 9 und der Applikationsstelle, her. Der Pfeil 23 gibt die Richtung des Gasstromes zur Applikationsstelle an.

Das Gerät arbeitet wie folgt :

Solange die Magnetspule 18 des aktiven 3/2-Weg-Ventils 1 von Strom durchflossen wird, kann Gas von der Druckgasquelle aus der Richtung 16 über die Anschlüsse 4 und 6 des Ventils 1, das die Ventile 1 und 2 verbindende Teilstück der Gaszuführungsleitung 3, und weiter über die Anschlüsse 7 und 8 des passiven 3/2-Weg-Schnellentlüftungsventils 2 in den Druckkessel 9 strömen. Der Dichtkörper 22 vom Ventil 2 wird dabei gegen den Ventilsitz 21 gepreßt. Sobald der Stromfluß durch die Magnetspule 18 unterbrochen wird, herrscht auf der dem Ventilsitz 20 zugekehrten Seite des Dichtkörpers 22 ein geringerer Druck als auf der über den Anschluß 8 dem Innendruck des nunmehr gefüllten Druckkessels 9 ausgesetzten anderen Seite des Dichtkörpers 22, weil das Ventil 1 die Verbindung zur Druckgasquelle sperrt und der Anschluß 7 des Ventils 2 über die Anschlüsse 5 und 6 des Ventils 1 mit der Umgebungsatmosphäre bzw. mit dem Inneren eines unter niedrigem Druck stehenden Behälters in Verbindung steht. Der Dichtkörper 22 wird dementsprechend gegen den Ventilsitz 20 gepreßt und der Druckkessel 9 entleert sich über die Anschlüsse 8 und 10 des passiven Ventils 2 in Richtung 23 zur Applikationsstelle hin.

Bei dem erläuterten Umschaltvorgang des passiven Ventils 2 geht auch ein gewisser Anteil des im Druckkessel 9 gespeicherten Gasvolumens infolge der Trägheit des Dichtkörpers 22 über den Anschluß 5 des aktiven Ventils 1 in Richtung 24 verloren. Dieser Anteil läßt sich durch Maßnahmen, welche die Ausströmungsgeschwindigkeit des Gases aus dem Anschluß 5 verringern — beispielsweise durch Vorsehen einer Querschnittsverengung mit Drosselwirkung — vermindern.

Wird das aktive 3/2-Weg-Ventil 1 sodann durch Stromzufuhr zur Magnetspule 18 wieder für die Gasdurchströmung von der Druckgasquelle geöffnet, wird das Ventil 2 wieder passiv umgeschaltet, die Entleerung des Druckkessels 9 zur Applikationsstelle wird unterbrochen und eine erneute Füllungsphase des Druckkessels beginnt.

Die voranstehend dargelegten Schaltvorgänge sind dem Schaltschema nach Fig. 2 in Kurzform entnehmbar. Die Schaltsymbole wurden — soweit möglich — durch die Bezugszahlen ergänzt, welche korrespondierenden Geräteteilen gemäß Fig. 1 zugeordnet sind. Die beiden Schaltstellungen des aktiven 3/2-Weg-Ventils 1 sind mit I und II gekennzeichnet.

Die Druckzunahme im Druckkessel verläuft, wie bereits weiter oben erwähnt, nicht linear. Der individuelle Verlauf der Druck-Zeit-Kennlinie hängt vom an der Druckgasquelle eingestellten Vordruck, von der Länge und dem Querschnitt der Gasleitung, von den Strömungsverhältnissen in den Ventilen, vom Volumen des Druckkessels 9 sowie vom zu erreichenden Solldruck im Druckkessel ab ; die grundsätzliche Form entspricht jedoch immer dem in Fig. 3 für einen Einzelfüllungstakt gezeigten Diagramm, in dem auf der Abszisse die Füllzeit und auf der Ordinate der zugeordnete Druck aufgetragen ist. Die Gaszufuhr beginnt zum Zeitpunkt $t_1$. Der Druck im Druckkessel nimmt zunächst schnell zu, dann verflacht die Kurve progressiv bis zur Einstellung des Maximaldruckes, welcher dem an der Druckgasquelle eingestellten Vordruck entspricht.

In der Praxis, d. h. wenn von dem Gerät höherfrequente Druckstöße abgegeben werden, erreicht der Druckkessel niemals den maximalen Füllungsgrad, die Entleerung beginnt bereits früher. Der Druckkessel wird jedoch auch nicht vollständig entleert, bevor der nächste Füllungstakt beginnt. Der tatsächliche Druckverlauf im Druckkessel, während das Gerät in Betrieb ist, ist aus Fig. 4 ersichtlich. Die Ventilumschaltung erfolgt mit konstanter Frequenz $1/\Delta t$ und der Kesseldruck verläuft zwischen den Grenzwerten $p_1$ und $p_2$. Der theoretische Kurvenverlauf ohne Ventilumschaltung ist für die Füllung und Entleerung durch strichlierte Linienzüge angedeutet. Zur Einstellung und Überwachung eines bestimmten Solldruckes ($p_2$ in Fig. 4) ist nach einer vorteilhaften Ausführungsform ein Druckfühler im Druckkessel 9 eingebaut, welcher über ein elektronisches Steuerelement den Umschaltzeitpunkt des aktiven 3/2-Weg-Ventils 1 genau festlegt.

Da es aber — wie bereits eingangs erwähnt — in Abhängigkeit vom gewünschten Solldruck zu einer unterschiedlichen Druckvoreilung ($\Delta_1 p$ und $\Delta_2 p$ gemäß Fig. 3) zwischen der Druckerfassung durch den Druckfühler und dem tatsächlichen Ansprechen der beiden 3/2-Weg-Ventile kommt (bei konstanter Zeitverzögerung $\Delta t$ gemäß Fig. 3), ist es besonders zweckmäßig, als Bestandteil des elektronischen Steuerelementes einen Mikroprozessor vorzusehen, welcher diese Druckcharakteristik kompensiert und das Signal zum Umschalten des aktiven 3/2-Weg-Ventils 1 bereits bei einem Druckwert, welcher sich aus dem Solldruck minus der jeweiligen Druckvoreilung $\Delta p$ ergibt, abgibt.

Für die klinische Praxis können die Ventile 1 und 2 einschließlich des verbindenden Leitungsstückes sowie des Druckkessels 9 in einem Gehäuse eingebaut sein, an welchem Gehäuse Außenanschlüsse für das von der Druckgasquelle kommende Leitungsstück und für das zur Applikationsstelle führende Leitungsstück vorgesehen sind. Diese Anschlüsse sind beispielsweise als konische Aufnahmen für einen Dichtkonus ausgebildet und mit Bajonettverschlüssen versehen. Die freiliegenden Leitungsstücke sind zweckmäßig flexible Kunststoffschläuche aus gegen reinen Sauerstoff beständigem Material, wie Polyurethan.

In Fig. 5 ist ein flexibles Leitungsstück 3, welches auf einen mit Bajonettverschluß versehenen Anschlußkonus 25 aufsteckbar ist, dargestellt. An das andere Ende des Leitungsstückes 3 ist eine nach dem Injektorprinzip arbeitende, allgemein mit 26 bezeichnete Befeuchtungsvorrichtung mit Flüssigkeitsvorratsbehälter 27 anschließbar. In die im Behälter 27 befindliche Befeuchtungsflüssigkeit taucht die Mündung 28 des Ansaugrohres 29, wenn der Vorratsbehälter 27 mit der Befeuchtungsvorrichtung 26 — im dargestellten Beispiel durch Aufstecken und Einrasten — vereinigt ist. An die Befeuchtungsvorrichtung 26 ist auch ein Konus 30 größeren Durchmessers angeformt, auf welchen ein Mundstück 31 oder ein geeignetes Verbindungsstück mit Intubierungsschlauch aufgeschoben werden kann. Unmittelbar am Ausgang 10 des Ventils 2 haben die Druckstöße noch ungefähr Dreiecksform, welche sich schon nach etwa 1 m Leitung eines Innendurchmessers von ca. 3 mm zwischen dem Ventil 2 und der Applikationsstelle weitgehend der erwünschten Sinushalbwelle annähert.

Die in Fig. 6 vergrößert dargestellte Mündung 28 des Ansaugrohres 29 weist ein gegen die Innenwand des Ansaugrohres abgedichtetes Kapillarrohr 32 auf.

Im Schaltplan nach Fig. 7 ist der Bauteil links von der strichpunktierten Linie identisch mit dem in Fig. 2 dargestellten. Zur Beimischung eines zweiten Gases ist ein weiteres aktives 3/2-Weg-Ventil 33 in die Gaszuführungsleitung 3 eingeschaltet, dessen Eingänge 4' und 5' jeweils mit einer Druckgasquelle, beispielsweise einer für Sauerstoff und einer für Luft, verbunden sind. Über den Anschluß 6' strömt abwechselnd eines der beiden Gase zu einem Reduzierventil 34, welches zwischen den beiden aktiven 3/2-Weg-Ventilen 1 und 33 in der Gaszuführungsleitung 3 eingeschaltet ist. Vom Ausgang des Reduzierventils 34 gelangt das Gas zum Anschluß 4 des aktiven 3/2-Weg-Ventils 1. Als weiteres aktives 3/2-Weg-Ventil 33 kommt zweckmäßigerweise ein elektropneumatisch gesteuertes 3/2-Weg-Ventil (dessen Schaltsymbol in Fig. 7 für das Ventil 33 verwendet 5 wurde) in Frage, da es nicht mit so hohen Frequenzen betrieben wird wie das erste aktive 3/2-Weg-Ventil 1.

Es ist weiters von Vorteil, ein Reduzierventil 34 mit justierbarem Ausgangsdruck einzubauen. Das Schaltsymbol für ein Ventil dieses Typs wurde in Fig. 7 verwendet. Ein elektronisches Steuerglied 35 dient dazu, die Umschaltung des weiteren aktiven 3/2-Weg-Ventils 33 vom Eingang 4' auf den Eingang 5' und umgekehrt nur in Schaltstellung I des ersten aktiven 3/2-Weg-Ventils 1 — wenn also die Ventile 33 und 34 nicht durchströmt sind — zuzulassen.

Bei der Ausführungsform nach Fig. 8 ist als aktives 3/2-Weg-Ventil 1 ein pneumatisches 3/2-Weg-Ventil in die Gaszuführungsleitung 3 eingeschaltet. Die den Anschlüssen des Magnetventiles gemäß Fig. 2 entsprechenden Anschlüsse dieses pneumatischen Ventiles sind mit gleichen Bezugszeichen wie in Fig. 2 versehen. Eine Zweigleitung 36 verbindet die Gaszuführungsleitung 3 und somit die Druckgasquelle mit dem Anschluß 37 eines weiteren aktiven pneumatischen 3/2-Weg-Ventiles 38, dessen zweiter Anschluß 39 in einen unter niedrigem Druck stehenden Behälter oder direkt in die Umgebungsluft mündet. Der dritte Anschluß 40 des Ventiles 38 ist mit dem Steuereingang 41 des pneumatischen 3/2-Weg-Ventiles 1 verbunden. Der Steuereingang 42 des weiteren pneumatischen Ventiles 38 ist mittels einer Gasleitung 43, in welche ein regelbares Drosselventil 44 geschaltet ist, an jenen Abschnitt der Gaszuführungsleitung 3 angeschlossen, welcher sich zwischen dem Anschluß 6 des aktiven 3/2-Weg-Ventiles 1 und dem Anschluß 7 des passiven 3/2-Weg-Ventiles 2 befindet.

Das passive 3/2-Weg-Ventil 2 sowie der Druckkessel 9 sind identisch mit den in Fig. 1 bzw. 2 dargestellten korrespondierenden Geräteteilen. Das weitere pneumatische 3/2-Weg-Ventil 38 kann auch direkt an die Druckgasquelle angeschlossen sein, in welchem Fall somit keine von der Gaszuführungsleitung 3 wegführende Zweigleitung 36, sondern eine zusätzliche direkte Verbindungsleitung vorzusehen ist.

Selbstverständlich ist es auch bei einem erfindungsgemäßen Gerät mit pneumatischem Ventil als aktivem 3/2-Weg-Ventil möglich, zur Beimischung eines zweiten Gases eine Kombination aus einem weiteren aktiven 3/2-Weg-Ventil und einem Reduzierventil zwischen die Druckgasquellen und das aktive 3/2-Weg-Ventil 1 — beispielsweise in der aus Fig. 7 ersichtlichen Art — einzuschalten.

Bei einem Gerät gemäß Fig. 8 ist das pneumatische 3/2-Weg-Ventil 1 ohne Druckausübung auf den Steuereingang 41 unter Wirkung der Feder 15 für die Gasströmung über den Anschluß 4 nach Anschluß 6 offen. Das Gas kann weiter über die Anschlüsse 7 und 8 des passiven 3/2-Weg-Ventiles in den Druckkessel 9 strömen. Die Durchströmung des weiteren pneumatischen 3/2-Weg-Ventiles 38 ist zunächst nicht möglich, da bei letzterem ohne Druckausübung auf seinen Steuereingang 42 die Verbindung zwischen den Anschlüssen 37 und 40 unterbrochen ist. Ein Teil des durch die Ventile 1 und 2 in den Druckkessel 9 strömenden Gases gelangt durch die Gasleitung 43 zum Steuereingang 42 des weiteren pneumatischen 3/2-Weg-Ventiles 38. Bei Erreichen eines bestimmten Druckes schaltet das Ventil 38 um.

Die Zeit, welche zum Erreichen dieses bestimmten Druckes benötigt wird, kann durch Verstellen des regelbaren Drosselventiles 44 vorgewählt werden. Beim Umschalten des Ventiles 38 wird dem Gas aus der Zweigleitung 36 der Weg zum Steuereingang 41 des Ventiles 1 freigegeben, wodurch das Ventil 1 mit nur sehr geringer Zeitverzögerung gegenüber dem Ventil 38 umschaltet.

Die Gasleitung 43 sowie der zwischen dem Ventil 1 und dem 15 Ventil 2 befindliche Abschnitt der Gaszuführungsleitung 3 werden dementsprechend drucklos, da das Gas über die Anschlüsse 6 und 5 des Ventiles 1 abströmt und das passive 3/2-Weg-Ventil 2 unter Entleerung des Druckkessels 9 über die Anschlüsse 8 und 10 zur Applikationsstelle umschaltet. Da sodann nach einer von der Einstellung des Drosselventils 44 abhängigen kurzen Zeitspanne am Steuereingang 42 des Ventiles 38 kein Druck mehr anliegt, schaltet das Ventil 38 wieder um, der Steuereingang 41 des Ventiles 1 wird über die Anschlüsse 40 und 39 des Ventiles 38 sehr schnell entlastet, dadurch wird in weiterer Folge auch das Umschalten des Ventiles 1 ausgelöst und das Auffüllen des Druckkessels 9 beginnt erneut.

Auf das Einschalten eines Drosselventiles 44 in die Gasleitung 43 kann verzichtet werden, wenn als weiteres pneumatisches 3/2-Weg-Ventil 38 ein solches gewählt wird, dessen Steuereingang mit einstellbarem Schaltpunkt ausgestattet ist, d. h. dessen Umschaltdruck veränderbar ist.

Wird ein erfindungsgemäßes Gerät zur Behandlung Frühgeborener, deren Atemzugsvolumen nur etwa 5 bis 15 ml beträgt und deren unreife Lungen äußerst empfindlich sind, eingesetzt, so kann zwischen dem aktiven 3/2-Weg-Ventil und der Druckgasquelle noch ein zusätzliches aktives 2/2-Weg-Ventil in der Gaszuführungsleitung vorgesehen werden. Werden hierfür Magnetventile verwendet, muß beispielsweise das 2/2-Weg-Ventil stromlos offen und das 3/2-Weg-Ventil stromlos geschlossen ausgeführt sein, d. h. das 2/2-Weg-Ventil schließt elektromagnetisch und das 3/2-Weg-Ventil ist für seine Schließfunktion auf Federkraft angewiesen. Die beiden Ventile werden mit zueinander invertierten elektrischen Signalen angesteuert. Da zum Zweck der Frühgeborenenbehandlung die einzelnen Geräteteile sehr klein ausgelegt sein müssen, ist auch das passive 3/2-Weg-Ventil dementsprechend klein und dessen Dichtkörper vergleichsweise rißanfällig.

Ein solches erfindungsgemäßes Gerät mit zusätzlichem aktivem 2/2-Weg-Ventil bietet noch weiter erhöhte Sicherheit. Sowohl bei Funktionsstörung « stromlos » als auch bei Funktionsstörung « Dauerstrom » ist immer ein aktives Ventil sicher geschlossen, und es kommt zu keiner länger dauernden Belastung des Dichtkörpers im passiven 3/2-Weg-Ventil, wodurch eventuelle Defekte vermieden werden.

**Patentansprüche**

1. Gerät zur Lungenbehandlung von Menschen oder Tieren, mit einer Druckgasquelle und einer zur Applikationsstelle führenden Leitung (3), in welche Gaszuführungsleitung (3) ein mit Frequenzen von 1 bis 20 Hz betätigbares aktives 3/2-Weg-Ventil (1) zur Bildung von Druckstößen im Bereich von 0,1 bis 5 bar eingeschaltet ist, mit einem zwischen diesem Ventil (1) und der Applikationsstelle weiters vorgesehenen passiven 3/2-Weg-Ventil (2), welches einen zwischen zwei Ventilsitzen (20, 21) hin- und herbewegbaren Dichtkörper (22) in der Leitung (3) aufweist, wobei in der einen Schaltstellung des aktiven 3/2-Weg-Ventils (1) eine Verbindung zwischen der Druckgasquelle und einem an den dritten Anschluß (8) des passiven 3/2-Weg-Ventils (2) angeschlossenen Druckkessel (9) herstellbar ist und der Dichtkörper (22) hierbei die Leitung zur Applikationsstelle sperrt und wobei in der anderen Schaltstellung des aktiven Ventils (1) durch Unterbrechung des Gasstromes von der Druckgasquelle eine Verbindung zwischen dem Druckkessel (9) und der Applikationsstelle herstellbar ist und der Dichtkörper (22) hierbei die Leitung zur Applikationsstelle freigibt, und gegebenenfalls mit einer vor der Applikationsstelle am Ende der Gaszuführungsleitung (3) angeordneten, nach dem Injektorprinzip arbeitenden Befeuchtungsvorrichtung (26).

2. Gerät nach Anspruch 1, dadurch gekennzeichnet, daß als aktives 3/2-Weg-Ventil (1) ein Magnetventil mit einem Dichtkolben (11) von geringer Masse und als passives 3/2-Weg-Ventil (2) ein Schnellentlüftungsventil mit im Vergleich zu dem aktiven 3/2-Weg-Ventil (1) größeren Strömungsquerschnitten vorgesehen sind.

3. Gerät nach Anspruch 1, dadurch gekennzeichnet, daß als aktives 3/2-Weg-Ventil (1) ein pneumatisches Ventil vorgesehen ist, dessen Steuereingang (41) über ein weiteres aktives pneumatisches 3/2-Weg-Ventil (38) mit der Druckgasquelle verbindbar ist, wobei der Steuereingang (42) des weiteren aktiven pneumatischen 3/2-Weg-Ventiles (38) mittels einer Gasleitung (43), in der sich gegebenenfalls ein regelbares Drosselorgan (44) befindet, zwischen dem aktiven 3/2-Weg-Ventil (1) und dem passiven 3/2-Weg-Ventil (2) an die Gaszuführungsleitung (3) angeschlossen ist.

4. Gerät nach Anspruch 3, dadurch gekennzeichnet, daß als passives 3/2-Weg-Ventil (2) ein Schnellentlüftungsventil mit im Vergleich zu den beiden aktiven pneumatischen 3/2-Weg-Ventilen (1 ; 38) größeren Strömungsquerschnitten vorgesehen ist.

5. Gerät nach einem der Ansprüche 1 bis 4, dadurch gekennzeichnet, daß das Ansaugrohr (29) der Befeuchtungsvorrichtung (26) einen etwa gleichen inneren Durchmesser hat wie die Gaszuführungsleitung (3), jedoch im Bereich der Mündung (28) in die Befeuchtungsflüssigkeit eine Drosselstelle, insbesondere ein gegen die Innenwand des Ansaugrohres (29) abgedichtetes Kapillarrohr (32), aufweist.

6. Gerät nach einem oder mehreren der Ansprüche 1 bis 5, dadurch gekennzeichnet, daß im Druckkessel (9) ein Druckfühler eingebaut ist, welcher über ein elektronisches Steuerelement elektrisch leitend mit dem aktiven 3/2-Weg-Ventil (1) verbunden ist.

7. Gerät nach Anspruch 6, dadurch gekennzeichnet, daß das elektronische Steuerelement einen Mikroprozessor zur exakten Regelung des Umschaltzeitpunktes des aktiven 3/2-Weg-Ventils unter Berücksichtigung der in Abhängigkeit vom Solldruck im Druckkessel veränderlichen Druckzunahme während der konstanten Zeitspanne zwischen der Druckerfassung durch den Druckfühler und dem Ansprechen der beiden 3/2-Weg-Ventile (1, 2) umfaßt.

8. Gerät nach einem oder beiden der Ansprüche 6 und 7, dadurch gekennzeichnet, daß als Druckfühler ein piezoelektrischer Druckwandler eingebaut ist.

9. Gerät nach einem oder mehreren der Ansprüche 1 bis 8, dadurch gekennzeichnet, daß zur Beimischung eines zweiten Gases in der Gaszuführungsleitung (3) in Richtung der Gasströmung gesehen vor dem aktiven 3/2-Weg-Ventil (1) ein weiteres aktives 3/2-Weg-Ventil (33) und zwischen den beiden aktiven 3/2-Weg-Ventilen (1 und 33) ein Reduzierventil (34) eingeschaltet sind, wobei die beiden Eingänge (4', 5') des weiteren aktiven 3/2-Weg-Ventils (33) jeweils mit einer Druckgasquelle verbunden sind.

10. Gerät nach Anspruch 9, dadurch gekennzeichnet, daß die beiden aktiven 3/2-Weg-Ventile (1, 33) über ein elektronisches Steuerglied (35) miteinander elektrisch verbunden sind, welches eine Umschaltung des weiteren aktiven 3/2-Weg-Ventils (33) nur zuläßt, solange das erste aktive 3/2-Weg-Ventil (1) die Gasströmung vom Reduzierventil (34) unterbricht.

**Claims**

1. Apparatus for treating the lungs of humans or animals including a source of pressure gas and a duct (3) leading to the site of application, into which gas supply duct (3) an active 3/2-way valve (1) to be actuated with frequencies of from 1 to 20 Hz is interposed for generating pressure shocks in the region of from 0.1 to 5 bar, a passive 3/2-way valve (2) further provided between that valve (1) and the site of application, which passive 3/2-way valve includes a sealing body (22) movable to and for between two valve seats (20, 21) in the duct (3), wherein in the one switching position of the active 3/2-way valve (1) a connection can be established between the source of pressure gas and a pressure vessel (9) connected to the third connection (8) of the passive 3/2-way valve (2), and the sealing body (22) in this case blocks the duct to the site of application, and wherein in the other switching position of the active valve (1), by interrupting the gas flow from the source of pressure gas, a connection can be established between the pressure vessel (9) and the site of application, and the sealing body (22) in this case clears the duct to the site of application, and, if desired, including a humidifying device (26) arranged in front of the site of application at the end of the gas supply duct (3) and working according to the injector principle.

2. Apparatus according to claim 1, characterised in that as the active 3/2-way valve (1) a solenoid valve having a sealing piston (11) of low mass, and as the passive 3/2-way valve (2) a rapid deaerating valve having larger flow cross-sections as compared to the active 3/2-way valve (1) are provided.

3. Apparatus according to claim 1, characterised in that as the active 3/2-way valve (1) a pneumatic valve is provided, whose control means (41) is connectable with the source of pressure gas via a further active pneumatic 3/2-way valve (38), wherein the control means (42) of the further active pneumatic 3/2-way valve (38), by means of a gas duct (43), in which, if desired, a controllable throttling organ (44) is provided, is connected to the gas supply duct (3) between the active 3/2-way valve (1) and the passive 3/2-way valve (2).

4. Apparatus according to claim 3, characterised in that as the passive 3/2-way valve (2) a rapid deaerating valve having larger flow cross-sections as compared to the two active pneumatic 3/2-way valves (1 ; 38) is provided.

5. Apparatus according to one of claims 1 to 4, characterised in that the suction pipe (29) of the humidifying device (26) has approximately the same inner diameter as the gas supply duct (3), yet includes a throttling site, in particular a capillary tube (32) sealed relative to the inner wall of the suction pipe (29), in the region of its entry (28) into the humidifying liquid.

6. Apparatus according to one or several of claims 1 to 5, characterised in that a pressure sensor is installed in the pressure vessel (9), which pressure sensor is electrically conductively connected with the active 3/2-way valve (1) via an electronic controlling element.

7. Apparatus according to claim 6, characterised in that the electronic controlling element includes a microprocessor for exactly regulating the switch-over time of the active 3/2-way valve taking into consideration a pressure increase variable in dependence on the nominal pressure prevailing in the pressure vessel over a constant span of time between pressure detection by the pressure sensor and response of the two 3/2-way valves (1, 2).

8. Apparatus according to one or both of claims 6 and 7, characterised in that a piezo-electric pressure transformer is installed as the pressure sensor.

9. Apparatus according to one or several of claims 1 to 8, characterised in that for admixing a second gas, a further active 3/2-way valve (33) is interposed in the gas supply duct (3) in front of the active 3/2-way valve, seen in the flow direction of the gas, and a reducing valve (34) is interposed between the two active 3/2-way valves (1 and 33), the two inlets (4', 5') of the further active 3/2-way valve (33) each being connected with a source of pressure gas.

10. Apparatus according to claim 9, characterised in that the two active 3/2-way valves (1, 33) are electrically interconnected via an electronic controlling member (35), which permits a switchover of the further active 3/2-way valve (33) only as long as the first active 3/2-way valve (1) interrupts the gas flow from the reducing valve (34).

## Revendications

1. Appareil pour le traitement des poumons de l'homme ou d'animaux, comprenant une source de gaz sous pression et une conduite (3) menant au point d'application et dans laquelle est incorporé un robinet actif (1) à 3 voies et 2 positions, qui peut être actionné avec des fréquences allant de 1 à 20 Hz en vue de la formation de pulsations de pression dans la plage de 0,1 à 5 bars, de même qu'un robinet passif (2) à 3 voies et 2 positions, prévu entre le robinet (1) et le point d'application et possédant un clapet (22) déplaçable en va-et-vient entre deux sièges (20, 21) dans la conduite (3), avec possibilité d'établissement, dans une position du robinet actif (1), d'une communication entre la source de gaz sous pression et une cuve de pression (9) raccordée au troisième orifice de raccordement (8) du robinet passif (2), pendant que le clapet (22) coupe la conduite menant au point d'application, et avec possibilité d'établissement, dans l'autre position du robinet actif (1), par suite de l'interruption du courant de gaz venant de la source de gaz sous pression, d'une communication entre la cuve de pression (9) et le point d'application, pendant que le clapet (22) permet la circulation de gaz par la conduite menant au point d'application, ainsi que, éventuellement, un humidificateur (26), travaillant selon le principe d'un injecteur, qui est disposé à l'extrémité de la conduite d'alimentation en gaz (3), avant le point d'application.

2. Appareil selon la revendication 1, caractérisé en ce que le robinet actif (1) est une électrovanne comportant un piston-obturateur (11) de faible masse et le robinet passif (2) est une soupape d'échappement rapide présentant des sections d'écoulement plus grandes que celles du robinet actif (1).

3. Appareil selon la revendication 1, caractérisé en ce que le robinet actif (1) est un robinet à commande pneumatique dont l'entrée de commande (41) peut être mise en communication avec la source de gaz sous pression par un robinet actif supplémentaire (38) à 3 voies et 2 positions et à commande pneumatique, l'entrée de commande (42) de ce robinet actif supplémentaire (38) étant raccordée à la conduite d'alimentation en gaz (3), entre le robinet actif (1) et le robinet passif (2), par une conduite de gaz (43) contenant éventuellement un organe d'étranglement réglable (44).

4. Appareil selon la revendication 3, caractérisé en ce que le robinet passif (2) est une soupape d'échappement rapide présentant des sections d'écoulement plus grandes que celles des deux robinets actifs (1 ; 38) à commande pneumatique.

5. Appareil selon une des revendications 1 à 4, caractérisé en ce que le tuyau d'aspiration (29) de l'humidificateur (26) possède à peu près le même diamètre intérieur que la conduite d'alimentation en gaz (3), mais présente un point d'étranglement, en particulier un tube capillaire (32) étanché par rapport à la paroi interne du tuyau d'aspiration (29), dans la région du bout (28) de ce tuyau plongeant dans le liquide d'humidification.

6. Appareil selon une ou plusieurs des revendications 1 à 5, caractérisé par l'incorporation dans la cuve de pression (9) d'un capteur de pression qui est connecté électriquement au robinet actif (1) à travers un élément de commande électronique.

7. Appareil selon la revendication 6, caractérisé en ce que l'élément de commande électronique comprend un microprocesseur pour le réglage exact de l'instant de commutation du robinet actif, en tenant compte de l'accroissement de pression, variable en fonction de la pression de consigne dans la cuve de pression, pendant l'intervalle de temps compris entre la détection de la pression par le capteur et la réponse des deux robinets (1, 2) à 3 voies et 2 positions.

8. Appareil selon une des revendications 6 et 7 ou selon les deux, caractérisé en ce que le capteur est un convertisseur de pression piézoélectrique.

9. Appareil selon une des revendications 1 à 8, caractérisé en ce que, pour l'addition en mélange d'un second gaz, un robinet actif supplémentaire (33), à 3 voies et 2 positions, est intercalé dans la conduite d'alimentation en gaz (3), avant le robinet actif (1) dans le sens de l'écoulement du gaz, et un détendeur (34) est intercalé entre les deux robinets actifs (1 et 33) à 3 voies et 2 positions, chacune des deux entrées (4', 5') du robinet actif supplémentaire (33) étant raccordée à une source de gaz sous pression.

10. Appareil selon la revendication 9, caractérisé en ce que les deux robinets actifs (1, 33) à 3 voies et 2 positions sont connectés électriquement entre eux par un organe de commande électronique (35) qui autorise seulement la commutation du robinet actif supplémentaire (33) tant que le premier robinet actif (1) coupe l'écoulement de gaz à partir du détendeur (34).

FIG. 1

FIG. 2

FIG. 3

FIG. 4

FIG. 7

FIG. 5

25

26

31

30

29

28

27

VI

FIG. 6

29

32

28

FIG. 8

42

38

40

37

39

43

37

44

39

40

36

41

5

6

4

9

1

5

2

4

8

6

3

3

43

7

15

10

3